# EUROPÄISCHE PATENTSCHRIFT

(11) **EP 3 164 067 B1**
(45) Veröffentlichungstag und Bekanntmachung des Hinweises auf die Patenterteilung: **22.05.2019**
(21) Anmeldenummer: 15739511.2
(22) Anmeldetag: 01.07.2015
(51) Int. Cl.: A61B 5/053, A61N 1/39

(54) **VORRICHTUNG FÜR EINEN IMPEDANZTOMOGRAPHEN**
DEVICE FOR AN IMPEDANCE TOMOGRAPH
DISPOSITIF POUR TOMOGRAPHE PAR IMPÉDANCE

(30) Priorität: 04.07.2014 DE 102014009890
(43) Veröffentlichungstag der Anmeldung: 10.05.2017
(73) Patentinhaber: Drägerwerk AG & Co. KGaA, 23558 Lübeck (DE)
(72) Erfinder: SATTLER, Frank, 23560 Lübeck (DE); HILTAWSKY, Karsten, 23617 Stockelsdorf (DE); LI, Jian-Hua, 23558 Lübeck (DE)
(74) Vertreter: Mildner, Volker
(86) Internationale Anmeldenummer: PCT/EP2015/001320
(87) Internationale Veröffentlichungsnummer: WO 2016/000821

(56) Entgegenhaltungen:
- EP-A1- 1 676 528
- DE-A1-102005 041 385
- US-A- 5 782 241
- CHANG-MING YANG ET AL: "Performance assessment of active electrode applied in wearable physiological monitoring system", BIOMEDICAL AND HEALTH INFORMATICS (BHI), 2012 IEEE-EMBS INTERNATIONAL CONFERENCE ON, IEEE, 5. Januar 2012 (2012-01-05), Seiten 472-474, XP032449106, DOI: 10.1109/BHI.2012.6211619 ISBN: 978-1-4577-2176-2

## Beschreibung

Die Erfindung betrifft eine Vorrichtung für einen elektrischen Impedanztomographen mit einem Elektrodenträger, einer ersten und einer zweiten Hautelektrode, die in Längsrichtung des Elektrodenträgers voneinander beabstandet an dem Elektrodenträger angeordnet sind, sowie einer ersten Schutzschaltung, die zumindest teilweise in den Elektrodenträger eingefasst ist.

Elektrodiagnostische Verfahren werden häufig an Patienten angewandt, die sich in einem kritischen Zustand befinden. Dabei kann es erforderlich werden, dass kurzfristig ein Defibrillator eingesetzt wird, ohne dass genügend Zeit besteht, Diagnosegeräte regulär vom Patienten zu trennen. Dadurch besteht die Gefahr einer Schädigung der Diagnosegeräte durch Überspannungspulse.

Die Defibrillation ist bei lebensbedrohlichen Situationen wie Kammerflimmern oder pulsloser Kammertachykardie die einzige effektive und lebenserhaltende Maßnahme.

Jede Verzögerung, die sich durch das Abnehmen von Elektroden oder elektrischen Anschlüssen vom Patienten ergeben würde, ist zu vermeiden.

Gemäß dem Stand der Technik verwendet man bei reinen EKG-Geräten oder bei kombinierten EKG-Impedanzmessgeräten, die nicht für bildgebende Verfahren verwendet werden, Eingangswiderstände im Bereich von 10-50 kOhm, um technische Schäden durch den Einsatz von Defibrillatoren zu verhindern.

Die besondere Schwierigkeit bei impedanztomographischen Verfahren besteht darin, dass bei Anwendungen in der thorakalen elektrischen Impedanztomographie im Gegensatz zur reinen Elektrokardiographie die Elektroden häufig für einen doppelten Einsatzzweck vorgesehen sind.

Sie sollen erstens die Anregungsströme, die bis zu 10 mA betragen können und mit denen eine gut auswertbare Potentialverteilung im Patienten erreicht werden soll, in den Patienten einleiten.

Zweitens sollen sie die geringen Signalspannungen, die aufgrund der mit den Anregungsströmen erzeugten Potentialverteilung an der Hautoberfläche des Patienten gemessen werden, dem Eingangsverstärker oder einer Auswerteeinheit wieder zuführen. Die zu messenden Signalspannungen können im Mikrovolt- bis Millivolt-Bereich liegen.

Die einzuleitenden Ströme müssen mit bis zu 10 mA hoch gewählt werden, um ausreichende Potentialunterschiede im gesamten Thorax zu erzeugen, um aus den abgegriffenen Potentialen ein Bild erzeugen zu können. Über Widerständen von 10-50 kOhm würden dabei Spannungen von 100-500 V abfallen. Solche Spannungen können am Patienten nicht zum Einsatz gebracht werden, weshalb die Möglichkeit der Absicherung des Impedanztomographen durch ausreichend hohe Schutzwiderstände nicht in Betracht zu ziehen ist.

Eine Absicherung der Eingänge durch parallel zum Eingangsverstärker geschaltete Varistoren oder Dioden ist ebenfalls problematisch. Zusätzliche Streukapazitäten, die zwischen Signalleitung und Bezugspotential geschaltet werden, müssen möglichst gering gehalten werden. Das ist erforderlich, damit bei den üblichen Arbeitsfrequenzen von etwa 10-200 kHz keine inakzeptablen Blindwiderstände entstehen, die parallel zum Eingang der ersten Verstärkerstufe liegen. Sie würden die Last erhöhen, die der Messkreis gegenüber der Potentialverteilung auf der Hautoberfläche des Patienten darstellt und könnten somit die Messung verfälschen. Bei einer Frequenz von 50 kHz stellen 10 pF bereits eine Impedanz von etwa 30 kOhm dar. Damit sind Lösungen nachteilig, die Varistoren oder Dioden parallel zum Eingangsverstärker beinhalten, wenn deren Störkapazität höher als einige pF ist. Dadurch scheiden aber alle Typen aus, die die Ströme, die üblicherweise durch einen Defibrillator-Schlag entstehen, dissipieren könnten.

Es muss daher davon ausgegangen werden, dass der Defibrillator zum Einsatz kommt, ohne dass der Patient vom Impedanztomographen dekonnektiert wird. Neben dem erforderlichen Schutz des Impedanztomographen vor einer Überlastung ergibt sich die Anforderung, eine zu starke Ableitung der Energie des Defibrillatorpulses zu vermeiden, um die Wirksamkeit des Defibrillators nicht unzulässig einzuschränken.

Beispielhaft fordern Normwerke, dass maximal 10% der Energie eines Defibrillatorpulses vom Messkreis dissipiert werden dürfen, wenn noch von einer ausreichenden Wirksamkeit ausgegangen werden soll.

Äquivalente normative Forderungen für thorakale Impedanztomographen sind im Falle einer Etablierung dieses diagnostischen Verfahrens zweifellos zu erwarten. Vor dem Schutz eventuell gefährdeter Gerätekomponenten steht die Gewährleistung eines wirkungsvollen Einsatzes des Defibrillators zum Schutze des Patienten.

Es bestand deshalb die Anforderung eine Vorrichtung bereitzustellen, die einen an einen Patienten angeschlossenen thorakalen elektrischen Impedanztomographen im Fall der Anwendung eines Defibrillators sicher vor Überspannungsschäden schützt, ohne dass die Gefahr besteht, dass die Energie der Defibrillatorpulse in einem Maße abgeleitet wird, die der Wirksamkeit der Defibrillation abträglich ist, wobei die Funktionsfähigkeit des Impedanztomographen erhalten bleiben soll.

Eine Vorrichtung zum Schutz eines elektrischen Impedanztomographen, die die zuvor genannte Anforderung erfüllt, ist beispielsweise aus der Druckschrift DE 10 2005 041 385 A1 bekannt. Offenbart wird darin ein elektrischer Impedanztomograph, dessen Signaleingänge mit einer Schutzschaltung versehen sind, die die Signaleingänge bei einem Anliegen einer für den normalen Messbetrieb zu hohen Spannung gegen zu hohe Eingangsströme sichert, wobei die Schutzschaltung in einem mit dem Impedanztomographen verwendeten Elektrodenträger integriert ist. Aus der US 5 782 241 A1, dem Artikel "Performance assessment of active electrode applied in wearable physiological monitoring system" von Chang-Ming Yang et al., Proc. IEEE-EMBS Conf. Biomed. Health Informatics (BHI 2012), 2.-7. Januar 2012, Seiten 472-474, sowie der EP 1 676 528 A1 sind diverse Vorrichtungen zum Detektieren von elektrokardiografischen Signalen bekannt.

In der Praxis werden für gewöhnlich Elektrodenträger mit einer Mehrzahl von Hautelektroden verwendet, die in einer Längsrichtung des Elektrodenträgers voneinander beabstandet an dem Elektrodenträger angeordnet sind. So können beispielsweise mindestens zwei, drei, fünf, zehn, fünfzehn oder mehr Elektroden vorgesehen sein. Wird nun die aus der zuvor genannten Druckschrift verwendete Vorrichtung zum Schutz des elektrischen Impedanztomographen verwendet, sind sämtliche Hautelektroden zunächst elektrisch mit der Schutzschaltung zu verbinden, um den in Signalleitungsrichtung nachgeordneten, elektrischen Impedanztomographen sicher vor Überspannungsschäden zu schützen. Die elektrischen Verbindungen zwischen der in den Elektrodenträger integrierten Schutzschaltung und der Vielzahl von Hautelektroden sind jeweils besonders elektrisch geschirmt, um zu verhindern, dass der Anwender den zuvor genannten hohen Spannungen, von etwa 100 Volt bis 500 Volt, ungewollt ausgesetzt ist. Mit anderen Worten sind die Hautelektroden durch besonders ausgebildete elektrische Leitungsverbindungen sternförmig mit der einen in den Elektrodenträger integrierten Schutzschaltung zu verbinden.

Bei der zuvor genannten sternförmigen Verbindungsart hat sich herausgestellt, dass entsprechende elektrische Leitungen oft sehr aufwendig und deshalb sehr kostenintensiv herzustellen sind. Denn neben dem vorzusehenden Schutz müssen derartigen Verbindungen auch eine hohe Biegsamkeit bieten, damit sich der Elektrodenträger möglichst abstandslos an den Körper des Patienten anlegen lässt.

Der Erfindung liegt deshalb die Aufgabe zugrunde, eine biegsame und möglichst kostengünstig herstellbare Vorrichtung für einen elektrischen Impedanztomographen mit einem Elektrodenträger und mehreren Hautelektroden bereitzustellen, die einen angeschlossenen elektrischen Impedanztomographen im Fall der Anwendung eines Defibrillators sicher vor Überspannungsschäden schützt.

Die Aufgabe wird gelöst durch eine Vorrichtung gemäß Anspruch 1.

Vorgesehen ist dazu eine Vorrichtung für einen elektrischen Impedanztomographen mit einem Elektrodenträger, einer ersten und einer zweiten Hautelektrode, die in Längsrichtung des Elektrodenträgers voneinander beabstandet an dem Elektrodenträger angeordnet sind, sowie einer ersten Schutzschaltung, die zumindest teilweise in den Elektrodenträger eingefasst ist, wobei die Vorrichtung eine zweite Schutzschaltung aufweist, die zumindest teilweise in den Elektrodenträger eingefasst ist, die erste Schutzschaltung mit der ersten Elektrode elektrisch verbunden ist und die zweite Schutzschaltung mit der zweiten Elektrode elektrisch verbunden ist, wobei die erste Schutzschaltung einen ersten Kondensator und die zweite Schutzschaltung einen zweiten Kondensator umfassen, wobei der erste und der zweite Kondensator zumindest einen Teil des Elektrodenträgers bilden.

Der Erfindung liegt die Erkenntnis zugrunde, dass eine zentrale Schutzschaltung einen verhältnismäßig großen Bauraum einnimmt, so dass bei einer Integration einer derartigen Schutzschaltung in den Elektrodenträger dieser seine notwendige Biegsamkeit verliert, um lückenlos an den Körper eines Patienten angelegt werden zu können. Darüber hinaus sind die elektrischen Verbindungsleitungen bei bekannten Vorrichtungen sternförmig zu der Mehrzahl der Elektroden ausgebildet. Diese Verbindungsleitungen müssen sowohl flexibel als auch elektrisch abschirmend und geschützt ausgebildet sein. Derartige elektrische Verbindungsleitungen sind nur mit einem sehr hohen Kostenaufwand herstellbar. Der Erfindung liegt deshalb der Gedanke zugrunde, mehrere Schutzschaltungen, insbesondere zwei Schutzschaltungen, vorzusehen, die jeweils mit unterschiedlichen Hautelektroden verbunden sind. Damit kann gewährleistet werden, dass die Schutzschaltungen elektrodennah angeordnet werden können. Die elektrischen Verbindungen zwischen den Schutzschaltungen und der jeweils mindestens einen zugehörigen Hautelektrode kann sehr kurz ausgestaltet werden. Damit verringern sich die Länge der Verbindungsleitungen sowie die Herstellungskosten. Darüber hinaus ist den erfindungsgemäßen Schutzschaltungen jeweils eine geringere Anzahl von Hautelektroden zugeordnet, so dass die Schutzschaltungen jeweils eine deutlich kleinere Bauform aufweisen. Sie können deshalb in den Elektrodenträger integriert werden, ohne dass dieser seine notwendige Biegsamkeit verliert.

Gemäß der erfindungsgemäßen Ausgestaltung sind mindestens zwei Schutzschaltungen vorgesehen, die jeweils in den Elektrodenträger eingefasst sind. Außerdem ist jede der Schutzschaltungen mit mindestens einer zugehörigen Hautelektrode elektrisch verbunden. Besonders bevorzugt ist jede der Schutzschaltungen benachbart zu ihrer zugehörigen Hautelektrode angeordnet. Die gewährleistet den besonders geringen Abstand zwischen der jeweiligen Schutzschaltung und der zugehörigen Hautelektrode.

Die erfindungsgemäße Ausgestaltung der Vorrichtung zeichnet sich weiterhin dadurch aus, dass die erste Schutzschaltung einen ersten Kondensator und die zweite Schutzschaltung einen zweiten Kondensator umfassen. Der als zweiter Kondensator bezeichnete Kondensator setzt nicht voraus, dass die zweite Schutzschaltung zwei oder mehr Kondensatoren aufweist. Vielmehr kann es nur der zweite Kondensator sein. Jede der Schutzschaltungen weist somit einen separaten Kondensator auf. Die Kondensatoren können somit an die entsprechenden Anforderungen angepasst werden. Außerdem weist jeder der Kondensatoren eine relativ kleine Baugröße auf, so dass die notwendige Biegsamkeit des Elektrodenträgen gewährleistet ist.

Eine weitere vorteilhafte Ausgestaltung der Vorrichtung zeichnet sich dadurch aus, dass eine der Schutzschaltungen in Längsrichtung zwischen der ersten und der zweiten Hautelektrode angeordnet ist. Erfindungsgemäß sind die erste und die zweite Hautelektrode an dem Elektrodenträger angeordnet. Damit soll auch erfasst sein, dass die Hautelektroden zumindest teilweise in den Elektrodenträger einfassen. Wäre eine Schutzschaltung nun oberhalb einer zugehörigen Hautelektrode angeordnet, würde sich die Dicke des Elektrodenträgers zumindest in diesem Bereich in der Regel erhöhen. In dem mindestens eine der Schutzschaltungen in Längsrichtung zwischen der ersten und der zweiten Hautelektrode angeordnet ist, kann der unerwünschte Effekt der Vergrößerung der Dicke des Elektrodenträges vermieden werden. Vielmehr ist es mit dieser Ausgestaltungsvariante möglich, die Dicke des Elektrodenträgers möglichst gering zu halten. Dies gewährleistet darüber hinaus eine gute Biegsamkeit. Besonders bevorzugt ist sowohl die erste Schutzschaltung als auch die zweite Schutzschaltung jeweils zwischen zwei Hautelektroden angeordnet. Somit kann der Elektrodenträger in Längsrichtung betrachtet eine möglichst geringe mittlere Dicke aufweisen.

Eine weitere vorteilhafte Ausgestaltung der Vorrichtung zeichnet sich dadurch aus, dass sich eine der Schutzschaltungen und eine der Hautelektroden in Längsrichtung überlappen. Diese Ausgestaltung ist insbesondere dann vorteilhaft, wenn die Schutzschaltung einen Bereich mit einer geringen Dicke und einen Bereich mit einer höheren Dicke ausweist. In diesem Fall kann die Schutzschaltung mit ihrem Bereich der höheren Dicke zwischen zwei Hautelektroden und mit ihrem Bereich der geringeren Dicke oberhalb einer der zuvor genannten Hautelektroden angeordnet sein. In diesem Fall überlappt die Schutzschaltung eine der Elektroden in Längsrichtung und gewährleistet trotzdem, dass der Elektrodenträger mit einer möglichst geringen Dicke ausgestaltet werden kann. Darüber hinaus kann es vorkommen, dass die Hautelektroden derart dicht benachbart zueinander angeordnet sind, dass die Schutzschaltungen nicht zwischen den Elektroden angeordnet werden können. In diesem Fall ist es sinnvoll, dass die mindestens eine Schutzschaltung derart in den Elektrodenträger eingefasst ist, dass sie eine der Hautelektroden in Längsrichtung überlappt. Sofern die Schutzschaltung mit mehreren Hautelektroden verbunden ist, kann es auch vorgesehen sein, dass die Schutzschaltung mehrere Hautelektroden überlappt.

Eine weitere vorteilhafte Ausgestaltung der Vorrichtung zeichnet sich dadurch aus, dass die erste und die zweite Schutzschaltung jeweils eine Schnittstelle zu einer Kontrolleinheit umfasst. Die Kontrolleinheit kann zur Datenvorverarbeitung und/oder zur Steuerung der mittels des Impedanztomographen einzuleitenden Ströme ausgebildet ist. Eine derartige Kontrolleinheit umfasst vorzugsweise aktive Bauelemente, die vor einer erhöhten Spannung, wie sie von einem Defibrillator abgegeben werden kann, zu schützen ist. Indem die Schutzschaltungen zwischen den Hautelektroden und der Kontrolleinheit angeordnet ist, wird die Kontrolleinheit von den Schutzschaltungen geschützt. Die Kontrolleinheit kann vorzugsweise ein integraler Bestandteil des Impedanztomographen sein. Alternativ kann es vorgesehen sein, dass die Kontrolleinheit an dem Elektrodenträger befestigt und/oder an diesem angeordnet ist. Besonders bevorzugt ist eine derartige Kontrolleinheit an einem Endabschnitt des Elektrodenträgers angeordnet, um die Biegsamkeit des Elektrodenträgers im Bereich der Elektroden weiterhin zu gewährleisten.

Eine weitere bevorzugte Ausgestaltung der Vorrichtung zeichnet sich dadurch aus, dass die erste und die zweite Hautelektrode lösbar an dem Elektrodenträger befestigt sind. Der Elektrodenträger und die Hautelektroden können somit nach der Verwendung separat voneinander gereinigt und/oder wiederaufbereitet werden. Außerdem bietet die getrennte Ausgestaltung die Möglichkeit, dass eine defekte Hautelektrode oder ein defekter Elektrodenträger ausgetauscht werden können und die übrigen Teile beibehalten werden.

Eine weitere vorteilhafte Ausgestaltung der Vorrichtung zeichnet sich dadurch aus, dass die erste und die zweite Hautelektrode in den Elektrodenträger integriert sind. Diese Ausgestaltung stellt sicher, dass eine Verbindung zwischen der Schutzschaltung und der zugehörigen Hautelektrode nicht ungewollt zugänglich ist. Vielmehr sind die Hautelektrode und die Schutzschaltung von dem Elektrodenträger bevorzugt eingekapselt. Dies bietet einen besonders sicheren Schutz für den Anwender und/oder den Patienten.

Eine nicht erfindungsgemäße Ausgestaltung der Vorrichtung zeichnet sich dadurch aus, dass der mindestens eine Kondensator als SMD ausgebildet ist. Mit SMD ist ein oberflächenmontiertes Bauelement gemeint. Somit kann der Kondensator unmittelbar auf einer Platine der zugehörigen Schutzschaltung angebracht sein, was eine besonders kleine Bauart für die zugehörige Schutzschaltung ermöglicht. Diese Schutzschaltung kann deshalb zwischen zwei Hautelektroden oder zu einer zugehörigen Hautelektrode überlappend angeordnet sein. Mit dem geringen Bauraum der Schutzschaltung kann darüber hinaus die notwendige Biegsamkeit des Elektrodenträgers gewährleistet bleiben.

Eine weitere vorteilhafte Ausgestaltung der Vorrichtung zeichnet sich dadurch aus, dass die mindestens zwei Kondensatoren als biegbare Kondensatoren ausgebildet sind. Somit sind der erste Kondensator und der zweite Kondensator jeweils als biegsamer Kondensator ausgebildet. Wird einer der Kondensatoren in den Elektrodenträger eingefasst oder von diesem umschlossen, bleibt der Elektrodenträger weiterhin biegsam. Der Elektrodenträger mit den daran angebrachten Hautelektroden kann deshalb besonders flexible und anpassbar an den Körper eines Patienten angelegt werden.

Eine weitere vorteilhafte Ausgestaltung der Vorrichtung zeichnet sich dadurch aus, dass ein Dielektrikum der mindestens zwei Kondensatoren als biegsame Folie ausgebildet ist. Weist einer der Kondensatoren darüber hinaus beispielsweise metallische Kondensatorelektroden auf, die eine entsprechende Biegsamkeit aufweisen, so ist auch der zugehörige Kondensator als solches biegsam ausgebildet. Deshalb weist ein derartiger Kondensator die Vorteile auf, wie sie zu der vorangegangenen, bevorzugten Ausgestaltung der Vorrichtung erläutert worden sind. Auf die entsprechenden Details und Vorteile wird deshalb Bezug genommen.

Eine weitere vorteilhafte Ausgestaltung der Vorrichtung zeichnet sich dadurch aus, dass die Kondensatorelektroden eines jeden Kondensators als biegsame Folie ausgebildet sind. Der Kondensator kann somit besonders einfach in den Elektrodenträger eingebracht werden. Darüber hinaus ist der Kondensator mit biegsamen Folien als Kondensatorelektroden und einer weiteren biegsamen Folie als Dielektrikum insgesamt als biegsamer Kondensator ausgebildet, der darüber hinaus besonders einfach in den Elektrodenträger eingefasst oder integriert werden kann.

Die erfindungsgemäße Ausgestaltung der Vorrichtung zeichnet sich dadurch aus, dass zumindest ein Teil des Elektrodenträgers von den mindestens zwei Kondensatoren gebildet ist. Auch wenn der Elektrodenträger vorzugsweise als biegsamer Elektrodenträger ausgebildet ist, hat sich eine bestimmte Festigkeit in der Praxis als vorteilhaft erwiesen. Wenn nun der Elektrodenträger zumindest teilweise von den Kondensatoren gebildet ist, können beispielsweise die Kondensatorelektroden die gewünschte Biegesteifigkeit und/oder Zugsteifigkeit des Elektrodenträgers mitbestimmen.

Eine weitere vorteilhafte Ausgestaltung der Vorrichtung zeichnet sich dadurch aus, dass der Elektrodenträger jeweils zumindest einen Teil eines Dielektrikums der mindestens zwei Kondensatoren bildet. Somit können der erste Kondensator und der zweite Kondensator in einem gemeinsamen Prozessschritt zur Herstellung des Elektrodenträgers hergestellt werden. Dies bietet darüber hinaus den Vorteil, dass die mindestens zwei Kondensatoren von dem Anwender von außen nicht zugänglich sind, sondern entsprechend abgeschirmt sind. Ein derartiger Elektrodenträger ist deshalb besonders sicher. Außerdem ist eine derartige Integration eines Kondensators in den Elektrodenträger besonders kompakt.

Weitere vorteilhafte Merkmale der Erfindung werden aus der Beschreibung der erfindungsgemäßen Ausführungsformen zusammen mit den Ansprüchen und/oder den beigefügten Zeichnung ersichtlich. Dabei können erfindungsgemäße Ausführungsformen einzelnen oder eine Kombination mehrerer Merkmale erfüllen. Die Erfindung wird nachstehend ohne Beschränkung des allgemeinen Erfindungsgedankens anhand von Ausführungsbeispielen unter Bezugnahme auf die Zeichnungen beschrieben. In den Zeichnungen zeigen:
- Figur 1: eine schematische Schnittdarstellung der erfindungsgemäßen Vorrichtung,
- Figur 2: eine schematische Schnittdarstellung eines Ausschnitts der erfindungsgemäßen Vorrichtung,
- Figur 3: eine schematische Draufsicht eines Ausschnitts der erfindungsgemäßen Vorrichtung und
- Figur 4: eine weitere schematische Schnittdarstellung eines Ausschnitts der erfindungsgemäßen Vorrichtung.

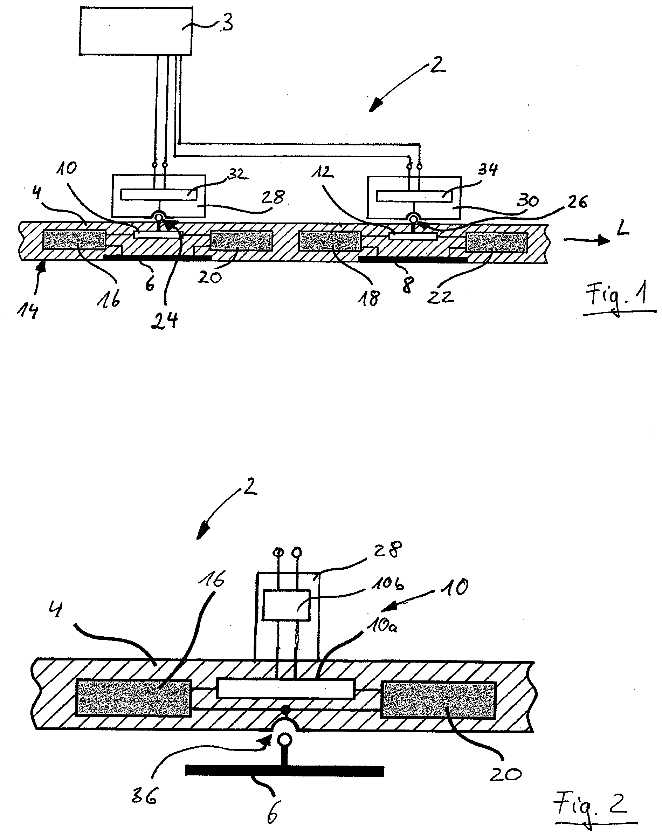

In der Figur 1 ist die erfindungsgemäße Vorrichtung 2 für einen elektrischen Impedanztomographen 3 gezeigt. Die Vorrichtung 2 umfasst dazu einen Elektrodenträger 4. Hierbei handelt es sich vorzugsweise um einen sog. Elektrodengürtel. Der Elektrodenträger 4 kann also wie ein Gürtel um den Körper eines Patienten gelegt werden. Um nun die für die Impedanztomographie notwendigen elektrische Signale an den Körper zu übermitteln oder elektrische Signale aufzunehmen, umfasst die Vorrichtung 2 eine erste Hautelektrode 6 und eine zweite Hautelektrode 8. Die erste und die zweite Hautelektrode 6, 8 sind in der Längsrichtung L des Elektrodenträgers 4 voneinander beabstandet an dem Elektrodenträger 4 angeordnet. Wird der Elektrodenträger 4 ringförmig gebogen, so sind die Hautelektroden 6, 8 radial innenseitig angeordnet. Die Hautelektroden 6, 8 kommen also unmittelbar mit dem Körper des Patienten in Kontakt und können somit die Übermittelung der gewünschten Signale gewährleisten. Wie aus der Figur 1 hervorgeht, können die Hautelektroden 6, 8 stetig mit einer Stirnfläche 14 des Elektrodenträgers 4 abschließen. Sie bilden sodann eine gemeinsame Oberfläche des Elektrodenträgers 4 bzw. der Vorrichtung 2.

In einer für einen Patienten kritischen Situation kann es vorkommen, dass der Impedanztomograph 3 und ein Defibrillator zeitgleich zur Anwendung kommen. Von dem Defibrillator werden hohe Spannungsimpulse ausgesendet. Um nun zu verhindern, dass die vom Defibrillator ausgesendeten Spannungsimpulse den Impedanztomographen 3 schädigen oder sogar zerstören, hat es sich als vorteilhaft erwiesen, eine erste Schutzschaltung 10 für die Vorrichtung 2 vorzusehen. Die Schutzschaltung 10 ist dabei zumindest teilweise in den Elektrodenträger 4 einzufassen. In der Praxis hat sich jedoch gezeigt, dass es von Vorteil ist, wenn die Schutzschaltung 10 vollständig oder zumindest ein erster Kondensator 16 der ersten Schutzschaltung 10 in den Elektrodenträger 4 eingefasst ist. Eine Ausgestaltung, bei der die gesamte erste Schutzschaltung 10 mit ihrem zugehörigen ersten Kondensator 16 in den Elektrodenträger 4 eingefasst ist, ist in der Figur 1 dargestellt.

Um den Elektrodenträger 4 um den Körper eines Patienten legen zu können, ist es notwendig, dass der Elektrodenträger 4 möglichst biegsam ausgebildet ist. Die gewünschte Biegsamkeit des Elektrodenträgers 4 wird gewährleistet, indem mindestens eine zweite Schutzschaltung 12 vorgesehen ist, die zumindest teilweise in den Elektrodenträger 4 eingefasst ist. Analog zu der ersten Schutzschaltung 10 umfasst die zweite Schutzschaltung 12 erfindungsgemäß einen zweiten Kondensator 18. An dieser Stelle sei erwähnt, dass mit dem ersten Kondensator 16 und dem zweiten Kondensator 18 jeweils ein Kondensator gemeint ist. Zwar können die erste Schutzschaltung 10 und die zweite Schutzschaltung 12 jeweils noch weitere Kondensatoren aufweisen, jedoch ist dies nicht zwingend notwendig.

Darüber hinaus ist es vorgesehen, dass die erste Schutzschaltung 10 mit der ersten Hautelektrode 6 elektrisch verbunden ist. Die zweite Schutzelektrode 12 ist mit der zweiten Hautelektrode 8 elektrisch verbunden. Vorzugsweise ist jede der Hautelektroden 6, 8 jeweils mit dem Kondensator 16, 18 der jeweils elektrisch verbundene Schutzschaltung 10, 12 unmittelbar elektrisch verbunden. Die Kopplung der ersten Schutzschaltung 10 mit der ersten Hautelektrode 6 und die Kopplung der zweiten Schutzschaltung 12 mit der zweiten Hautelektrode 8 bieten den Vorteil, dass der Schutz vor elektrischen Impulsen eines Defibrillator elektrodennah erfolgt. Da mehrere Schutzschaltungen 10, 12 vorgesehen sind, weisen sie jeweils eine relativ kleine Baugröße auf, so dass selbst bei einer Einfassung der Schutzschaltungen 10, 12 in den Elektrodenträger 4 die Biegsamkeit des Elektrodenträgers 4 erhalten bleibt. Um die Biegsamkeit des Elektrodenträgers 4 noch weiter zu begünstigen, hat es sich in der Praxis als vorteilhaft erwiesen, wenn die für eine Schutzschaltung 10, 12 vorgesehene elektrische Kapazität auf mehrere Kondensatoren aufgeteilt wird. So ist es beispielsweise von Vorteil, wenn die erste Schutzschaltung 10 den ersten Kondensator 16 und einen weiteren Kondensator 20 umfasst. Die beiden Kondensatoren 16, 20 können sodann die für die erste Schutzschaltung 10 gewünschte Kapazität bereitstellen. Entsprechend können für die zweite Schutzschaltung 12 der zweite Kondensator 18 und ein weiterer Kondensator 22 vorgesehen sein. Auch hier erfolgt eine Aufteilung der gewünschten Kapazität. Mit der Mehrzahl der Kondensatoren 16, 20, 18, 22 weist jeder der Kondensatoren 16, 20, 18, 22 eine kleinere Baugröße auf. Dies gewährleistet eine höhere Biegsamkeit des Elektrodenträgers 4.

Zu jeder der Schutzschaltungen 10, 12 ist ein zugehöriger Anschluss 24, 26 vorgesehen. Der erste Anschluss 24 ist mit der ersten Schutzschaltung 10 verbunden. Der zweite Anschluss 26 ist mit der zweiten Schutzschaltung 12 verbunden. Die Anschlüsse 24, 26 dienen zum Verbinden der Schutzschaltungen 10, 12 mit dem Impedanztomographen 3.

In der Praxis hat es sich als vorteilhaft erwiesen, wenn zwischen dem Impedanztomographen 3 und dem Elektrodenträger 4 eine Vorverarbeitungseinheit vorgesehen ist. Besonders bevorzugt ist jeder der Hautelektroden 6, 8 bzw. jeder der Schutzschaltungen 10, 12 eine Vorverarbeitungseinheit 28, 30 zugeordnet. So kann die erste Schutzschaltung 10 mit einer ersten Vorverarbeitungseinheit 28 verbunden sein. Die zweite Schutzschaltung 12 kann mit einer zweiten Vorverarbeitungseinheit 30 verbunden sein. Die beiden Vorverarbeitungseinheiten 28, 30 sind vorzugsweise mittels elektrischer Leitungen mit dem Impedanztomographen 3 verbunden. Mit den Vorverarbeitungseinheiten 28, 30 können die von den Hautelektroden 6, 8 erfassten Signale vorverarbeitet und/oder zumindest teilweise ausgewertet werden. Die Vorverarbeitungseinheiten 28, 30 können insbesondere zur Weiterleitung, Anpassung und/oder Verstärkung eines elektrischen Signals ausgebildet sein. Zur Erfüllung ihres jeweiligen Zwecks kann jede Vorverarbeitungseinheit 28, 30 eine Elektronik 32, 34 mit aktiven und/oder passiven elektrischen Bauelementen umfassen.

In der Figur 2 ist ein Ausschnitt der erfindungsgemäßen Vorrichtung 2 dargestellt, wobei dieser den Elektrodenträger 4, die erste Hautelektrode 6 und die erste Schutzschaltung 10 aufweist. Die erste Schutzschaltung 10 ist mit der ersten Hautelektrode 6 elektrische verbunden. Wenn im Folgenden Aspekte zu der ersten Hautelektrode 6 und/oder der ersten Schutzschaltung 10 erläutert werden, soll dies analog für die zweite Hautelektrode 8 bzw. die zweite Schutzschaltung 12 gelten.

Für die erste Hautelektrode 6 weist der Elektrodenträger 4 eine Aufnahme 36 auf. In diese Aufnahme 36 kann die erste Hautelektrode 6 eingesetzt werden und/oder von dieser befestigt werden. Somit ist es möglich, dass die erste Hautelektrode 6 lösbar an dem Elektrodenträger 4 befestigt ist. Zwischen der ersten Hautelektrode 6 und dem Elektrodenträger 4 bildet sich vorzugsweise eine Schnellbefestigung aus.

Außerdem ist aus der Figur 2 zu erkennen, dass die Schutzschaltung 10 mehrteilig ausgebildet ist. Sie weist in diesem Fall einen ersten Schutzschaltungsteil 10a und einen zweiten Schutzschaltungsteil 10b auf. Der erste Schutzschaltungsteil 10a ist in den Elektrodenträger 4 vollständig eingefasst. Der zweite Schutzschaltungsteil 10b ist der ersten Vorverarbeitungseinheit 28 zugeordnet. Verbunden sind die beiden Teile der Schutzschaltung 10 mittels entsprechender elektrischer Leitungen. Die erste Vorverarbeitungseinheit 28 kann fest oder lösbar an dem Elektrodenträger 4 befestigt sein. Bei einer lösbaren Befestigung der ersten Vorverarbeitungseinheit 28 können entsprechende Schnellbefestigungsmittel vorgesehen sein. Durch die Aufteilung der ersten Schutzschaltung 10 ist es möglich, den zur Verfügung stehenden Bauraum möglichst optimal auszunutzen. Außerdem können Bauteile der Schutzschaltung 10 in den ersten Schutzschaltungsteil 10a integriert sein, die einen Anwender des Impedanztomographen 3 oder einen Anwender des Defibrillators schützen. So kann der erste Schutzschaltungsteil 10a beispielsweise einen Hochspannungsschutz aufweisen. Weitere elektrische Bauteile der Schutzschaltung 10 können sodann von dem zweiten Schutzschaltungsteil 10b umfasst sein. Sollte die erste Vorverarbeitungseinheit 28 sodann lösbar an den Elektrodenträger 4 befestigt sein, wird der zuvor genannte Anwender davor geschützt, einem hohen Spannungsimpuls des Defibrillators unbeabsichtigt ausgesetzt zu sein.

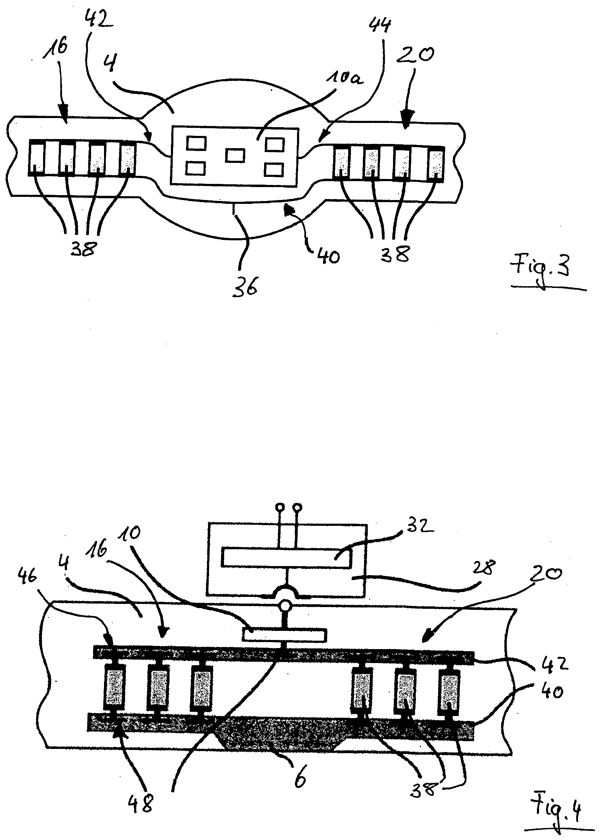

In der Figur 3 ist eine schematische Draufsicht des Ausschnitts der Vorrichtung 2 aus der Figur 2 zu erkennen. Hieraus wird ersichtlich, dass der erste Kondensator 16 und der weitere Kondensator 20 jeweils in eine Mehrzahl von Einzelkondensatoren 38unterteilt sind. Die Einzelkondensatoren 38 können parallel zueinander angeordnet sein, um den ersten Kondensator 16 bzw. den weiteren Kondensator 20 zu bilden. Besonders bevorzugt ist jeweils ein Anschluss eines jeden Einzelkondensators 38 mit einer gemeinsamen, elektrischen Verbindungsleitung 40 verbunden. An diese Verbindungsleitung 40 ist auch die Aufnahme 36 angeschlossen, um mit dieser die erste Hautelektrode 6 zu verbinden. Die verbleibenden Anschlüsse der Einzelkondensatoren 38 des ersten Kondensators 16 sind mittels einer weiteren Verbindungsleitung 42 verbunden. Diese weitere Verbindungsleitung 42 ist mit der übrigen ersten Schutzschaltungsteil 10a bzw. mit der übrigen ersten Schutzschaltung 10 verbunden. Für den weiteren Kondensator 20 ist in analoger Weise eine weitere Verbindungsleitung 44 vorgesehen, mit der die übrigen, dem weiteren Kondensator 20 zugehörigen Anschlüsse der Einzelkondensatoren 38 verbunden sind. Die weitere Verbindungsleitung 44 ist ebenfalls mit der übrigen ersten Schutzschaltungsteil 10a bzw. der übrigen ersten Schutzschaltung 10 verbunden. Mit dem zu Figur 3 erläuterten Aufbau ist es möglich, dass der Elektrodenträger 4 zumindest im Bereich zwischen den Hautelektroden 6, 8 biegsam ausgestaltet ist, um ein dichtes Anliegen des Elektrodenträgers 4 an dem Körper des Patienten zu ermöglichen.

Aus der Figur 4 gehen weitere Aspekte zu dem Aufbau der Vorrichtung 2 hervor. Analog zur Figur 2 wird auch hier darauf verwiesen, dass im Weiteren der Elektrodenträger 4 mit der ersten Schutzschaltung 10 und der ersten Hautelektrode 6 erörtert wird, wobei die genannten Merkmale und Vorteile in analoger Weise für die zweite Schutzschaltung 12 und die zweite Hautelektrode 8 gelten sollen.

Aus der Figur 4 ist zu erkennen, dass die weitere Verbindungsleitung 42 jeweils einen Anschluss 46 eines jeden Einzelkondensators 38 des ersten Kondensators 16 und des weiteren Kondensators 20 miteinander elektrisch verbindet. Die Einzelkondensatoren 38 sind mit ihrem jeweiligen weiteren Anschluss 48 mit einer Verbindungsleitung 40 verbunden. Diese Verbindungsleitung 40 ist dabei in der Weise ausgebildet, dass sie die erste Hautelektrode 6 bildet.

### BEZUGSZEICHENLISTE

### (Teil der Beschreibung)

- L: Längsrichtung
- 2: Vorrichtung
- 3: Impedanztomograph
- 4: Elektrodenträger
- 6: erste Hautelektrode
- 8: zweite Hautelektrode
- 10: erste Schutzschaltung
- 10a: erster Schutzschaltungsteil
- 10b: zweiter Schutzschaltungsteil
- 12: zweite Schutzschaltung
- 14: Stirnfläche
- 16: erster Kondensator
- 18: zweiter Kondensator
- 20: weiterer Kondensator
- 22: weiterer Kondensator
- 24: Anschluss
- 26: Anschluss
- 28: erste Vorverarbeitungseinheit
- 30: zweite Vorverarbeitungseinheit
- 32: Elektronik
- 34: Elektronik
- 36: Aufnahme
- 38: Einzelkondensator
- 40: Verbindungsleitung
- 42: Verbindungsleitung
- 44: Verbindungsleitung
- 46: Anschluss
- 48: Anschluss

## Patentansprüche

1. Vorrichtung (2) für einen elektrischen Impedanztomographen (3) mit
- einem Elektrodenträger (4),
- einer ersten und einer zweiten Hautelektrode (6, 8), die in Längsrichtung L des Elektrodenträgers (4) voneinander beabstandet an dem Elektrodenträger (4) angeordnet sind, sowie
- einer ersten Schutzschaltung (10), die zumindest teilweise in den Elektrodenträger (4) eingefasst ist, sowie
- eine zweite Schutzschaltung (12), die zumindest teilweise in den Elektrodenträger (4) eingefasst ist,
- wobei die erste Schutzschaltung (10) mit der ersten Hautelektrode (6) elektrisch verbunden ist und
- die zweite Schutzschaltung (12) mit der zweiten Hautelektrode (8) elektrisch verbunden ist,
**dadurch gekennzeichnet, dass**
die erste Schutzschaltung (10) einen ersten Kondensator (16) und die zweite Schutzschaltung (12) einen zweiten Kondensator (18) umfassen, und dass der erste und der zweite Kondensator (16, 18) zumindest einen Teil des Elektrodenträgers (4) bilden.

2. Vorrichtung (2) nach einem der vorhergehenden Ansprüche, **dadurch gekennzeichnet, dass** eine der Schutzschaltungen (10, 12) in Längsrichtung L zwischen der ersten und der zweiten Hautelektrode (6, 8) angeordnet ist.

3. Vorrichtung (2) nach einem der vorhergehenden Ansprüche, **dadurch gekennzeichnet, dass** sich eine der Schutzschaltungen (10, 12) und eine der Hautelektroden (6, 8) in Längsrichtung L überlappen.

4. Vorrichtung (2) nach einem der vorhergehenden Ansprüche, **dadurch gekennzeichnet, dass** die erste und die zweite Schutzschaltung (10, 12) jeweils eine Schnittstelle zu einer Kontrolleinheit umfasst.

5. Vorrichtung (2) nach Anspruch 4, **dadurch gekennzeichnet, dass** die Kontrolleinheit von dem Impedanztomographen (3) umfasst oder an dem Elektrodenträger (4) angeordnet ist.

6. Vorrichtung (2) nach einem der vorhergehenden Ansprüche, **dadurch gekennzeichnet, dass** die erste und die zweite Hautelektrode (6, 8) an dem Elektrodenträger (4) lösbar befestigt oder in den Elektrodenträger (4) integriert sind.

7. Vorrichtung (2) nach einem der vorhergehenden Ansprüche, **dadurch gekennzeichnet, dass** die mindestens zwei Kondensatoren (16, 18) als biegbare Kondensatoren ausgebildet sind.

8. Vorrichtung (2) nach einem der vorhergehenden Ansprüche, **dadurch gekennzeichnet, dass** ein Dielektrikum der mindestens zwei Kondensatoren (16, 18) als biegsame Folie ausgebildet ist.

9. Vorrichtung (2) nach einem der vorhergehenden Ansprüche, **dadurch gekennzeichnet, dass** die Kondensatorkontaktflächen eines jeden Kondensators (16, 18) als biegsame Folie ausgebildet sind.

10. Vorrichtung (2) nach einem der vorhergehenden Ansprüche, **dadurch gekennzeichnet, dass** der Elektrodenträger (4) zumindest einen Teil eines jeden Dielektrikums der mindestens zwei Kondensatoren (16, 18) bildet.

## Claims

1. Device (2) for an electrical impedance tomograph (3) having
- an electrode support (4),
- a first and a second skin electrode (6, 8), which are arranged on the electrode support (4) so as to be spaced apart from one another in the longitudinal direction L of the electrode support (4), and
- a first protective circuit (10) that is at least partly contained in the electrode support (4), and
- a second protective circuit (12) that is at least partly contained in the electrode support (4),
- wherein the first protective circuit (10) is electrically connected to the first skin electrode (6), and
- the second protective circuit (12) is electrically connected to the second skin electrode (8),
**characterized in that**
the first protective circuit (10) comprises a first capacitor (16) and the second protective circuit (12) comprises a second capacitor (18), and **in that** the first and the second capacitor (16, 18) form at least part of the electrode support (4).

2. Device (2) according to one of the preceding claims, **characterized in that** one of the protective circuits (10, 12) is arranged between the first and the second skin electrode (6, 8) in the longitudinal direction L.

3. Device (2) according to either of the preceding claims, **characterized in that** one of the protective circuits (10, 12) and one of the skin electrodes (6, 8) overlap in the longitudinal direction L.

4. Device (2) according to one of the preceding claims, **characterized in that** the first and the second protective circuit (10, 12) each comprise an interface to a control unit.

5. Device (2) according to Claim 4, **characterized in that** the control unit is contained in the impedance tomograph (3) or arranged on the electrode support (4).

6. Device (2) according to one of the preceding claims, **characterized in that** the first and the second skin electrode (6, 8) are releasably fastened to the electrode support (4) or integrated into the electrode support (4).

7. Device (2) according to one of the preceding claims, **characterized in that** the at least two capacitors (16, 18) are designed as flexible capacitors.

8. Device (2) according to one of the preceding claims, **characterized in that** a dielectric of the at least two capacitors (16, 18) is designed as a flexible film.

9. Device (2) according to one of the preceding claims, **characterized in that** the capacitor contact surfaces of a respective capacitor (16, 18) are designed as a flexible film.

10. Device (2) according to one of the preceding claims, **characterized in that** the electrode support (4) forms at least part of a respective dielectric of the at least two capacitors (16, 18).

## Revendications

1. Dispositif (2) pour un tomographe par impédance électrique (3) comprenant
- un porte-électrodes (4),
- une première et une deuxième électrode cutanée (6, 8) qui sont disposées sur le porte-électrodes (4) à distance l'une de l'autre dans la direction longitudinale L du porte-électrodes (4) et
- un premier circuit de protection (10) qui est au moins partiellement enchâssé dans le porte-électrodes (4),
ainsi que
- un deuxième circuit de protection (12) qui est au moins partiellement enchâssé dans le porte-électrodes (4),
- le premier circuit de protection (10) étant relié électriquement à la première électrode cutanée (6), et
- le deuxième circuit de protection (12) étant relié électriquement à la deuxième électrode cutanée (8),
**caractérisé en ce que**
le premier circuit de protection (10) comprend un premier condensateur (16) et le deuxième circuit de protection (12) comprend un deuxième condensateur (18), et **en ce que** les premier et deuxième condensateurs (16, 18) forment au moins une partie du porte-électrodes (4).

2. Dispositif (2) selon l'une des revendications précédentes, **caractérisé en ce que** l'un des circuits de protection (10, 12) est disposé entre la première et la deuxième électrode cutanée (6, 8) dans la direction longitudinale L.

3. Dispositif (2) selon l'une des revendications précédentes, **caractérisé en ce que** l'un des circuits de protection (10, 12) et l'une des électrodes cutanées (6, 8) se recouvrent dans la direction longitudinale L.

4. Dispositif (2) selon l'une des revendications précédentes, **caractérisé en ce que** les premier et deuxième circuits de protection (10, 12) comprennent chacun une interface avec une unité de commande.

5. Dispositif (2) selon la revendication 4, **caractérisé en ce que** l'unité de commande est comprise par le tomographe par impédance (3) ou disposée sur le porte-électrodes (4).

6. Dispositif (2) selon l'une des revendications précédentes, **caractérisé en ce que** les première et deuxième électrodes cutanées (6, 8) sont fixées de manière amovible sur le porte-électrodes (4) ou intégrées dans le porte-électrodes (4).

7. Dispositif (2) selon l'une des revendications précédentes, **caractérisé en ce que** lesdits au moins deux condensateurs (16, 18) sont réalisés sous la forme de condensateurs flexibles.

8. Dispositif (2) selon l'une des revendications précédentes, **caractérisé en ce qu'**un diélectrique desdits au moins deux condensateurs (16, 18) est réalisé sous la forme d'une feuille souple.

9. Dispositif (2) selon l'une des revendications précédentes, **caractérisé en ce que** les surfaces de contact de condensateur de chaque condensateur (16, 18) sont réalisées sous la forme d'une feuille souple.

10. Dispositif (2) selon l'une des revendications précédentes, **caractérisé en ce que** le porte-électrodes (4) forme au moins une partie de chaque diélectrique desdits au moins deux condensateurs (16, 18).
